# EUROPEAN PATENT SPECIFICATION

(11) **EP 4 547 342 B1**
(45) Date of publication and mention of the grant of the patent: **28.01.2026**
(21) Application number: 23731612.0
(22) Date of filing: 07.06.2023
(51) Int. Cl.: A61Q 19/10, A61K 8/34, A61K 8/365, A61K 8/44, A61K 8/46, A61K 8/67, C11D 1/10, C11D 3/34, A61K 8/73, A61Q 5/02

(54) **STABLE WASH COMPOSITION WITH UNSATURATED ZWITTERIONIC SURFACTANT**
STABILE WASCHMITTELZUSAMMENSETZUNG MIT EINEM UNGESÄTTIGTEN ZWITTERIONISCHEN TENSID
COMPOSITION DE LAVAGE STABLE COMPORTANT UN AGENT TENSIOACTIF ZWITTERIONIQUE INSATURÉ

(30) Priority: 30.06.2022 US 202263357387 P; 06.09.2022 EP 22194213
(43) Date of publication of application: 07.05.2025
(73) Proprietor: Unilever IP Holdings B.V., 6708 WH Wageningen (NL); Unilever Global IP Limited, Wirral, Merseyside CH62 4ZD (GB)
(72) Inventor: YUAN, Mingjun, 6708 WH Wageningen (NL); VASUDEVAN, Tirucherai, Varahan, 6708 WH Wageningen (NL)
(74) Representative: Askew, Sarah Elizabeth
(86) International application number: PCT/EP2023/065243
(87) International publication number: WO 2024/002647

(56) References cited:
- WO-A1-2020/233971
- WO-A1-2020/233972
- WO-A1-2021/084007
- WO-A1-98/56497
- CN-A- 111 973 522
- US-A1- 2016 095 804

## Description

### Field of the Invention

The present invention is directed to a stable wash composition comprising biodegradable thickener. More particularly, the invention is directed to a wash composition comprising water, anionic surfactant and biodegradable thickener used with unsaturated zwitterionic surfactant. Such a composition surprisingly yields excellent lather and viscosity characteristics, rinses easily, is free of syneresis, and maintains stable viscosity after being stored at elevated temperatures, even when formulated substantially free of soap and sulfates, and predominately free of synthetic thickeners like ammonium acryloyldimethyltaurate vinylpyrrolidone copolymer, alkyl acrylate cross polymers or the like.

### Background of the Invention

Wash compositions are typically employed to cleanse surfaces, like skin, and often to reduce shine associated with sebum produced in specialized epithelial cells known as sebocytes. They are also used to minimize bacteria on surfaces, like the hands and face, whereby washing is viewed as the most effective way to prevent the spread of germs and bacteria. In fact, experts believe that periodic washing throughout the day can reduce the number of consumers catching colds by about 50%.

Consumers know it is generally a good practice to regularly clean their hands and not touch their face, eyes and mouth in order to minimize the risk of getting sick. Information provided by the Centers for Disease Control and Prevention suggests that the COVID-19 (coronavirus) pandemic is a direct result of a virus that is more efficient and severe than influenza and that spreads rapidly from person to person via respiratory droplets. During such a pandemic, consumers understand and medical professionals emphasize it is in everyone's best interest to consistently wash their hands and face, and inanimate surfaces they come in contact with prior to use.

With consumers washing more often, it is highly desirable to develop wash compositions that are not only good for the consumer but enjoyable to use and friendlier towards the environment. This invention, therefore, is directed to a stable wash composition comprising water, anionic surfactant, biodegradable thickener and unsaturated zwitterionic surfactant. The wash composition surprisingly yields excellent lather and viscosity characteristics, is free of syneresis, and maintains stable viscosity after being stored at elevated temperatures, even when formulated substantially free of soap and sulfates, and predominately free of synthetic thickeners like ammonium acryloyldimethyltaurate vinylpyrrolidone copolymer, alkyl acrylate crosslinked polymers or the like.

### Additional Information

Efforts have been disclosed for making wash compositions. In U.S. Patent Application No. 2019/008738A1, personal care compositions with cationic surfactant, nonionic surfactant and a polyacrylate cross-polymer structuring agent are described. The wash compositions can include carrageenan as a thickener.

Other efforts have been disclosed for making wash compositions. In U.S. Patent Application No. 2019/0359735A1, water soluble hybrid polymers suitable for use in a body wash are described. Even other efforts have been disclosed for making wash compositions. In U.S. Patent No. 6,566,313, shampoo and body wash compositions having a conditioning complex formed from a tertiary alkyl amidoamine and a phosphate ester are described.

Still other efforts have been described for making wash compositions. In U.S. Patent No. 9,549,885, scalp care compositions that can include synthetic rheology modifiers like acrylamide/ammonium acrylates/C10-C30 alkyl acrylate crosslinked polymer or acrylates/steareth-20 itaconate copolymer are described.

Yet other efforts for making wash compositions are described. In U.S. Patent No. 7,375,063, cleansing compositions with an aqueous acrylate copolymer emulsion, amphoteric surfactant and anionic surfactant are described.

WO 2020/233971 A1 discloses isotropic liquid cleansing compositions comprising acyl isethionate, methyl acyl taurate and amphoteric, zwitterionic and/or nonionic surfactant. The compositions, at least in one embodiment, are preferably sulfate free and mild to the skin and scalp.

None of the additional information describes a composition with biodegradable thickener and unsaturated zwitterionic surfactant as claimed herein.

### Summary of the Invention

In a first aspect, the present invention is directed to a wash composition comprising:
a) at least one anionic surfactant;
b) unsaturated zwitterionic surfactant;
c) 0 to 2% by weight nonionic surfactant; and
d) thickener having starch, and at least one of cellulose and gum
wherein the unsaturated zwitterionic surfactant comprises a hydrophobic tail comprising 2.5 to 20%, and preferably, 3.5 to 15%, and most preferably, from 4 to 14% (or 4.5 to 13%) by weight C18:1, and 0.45 to 4.5%, and preferably, 0.75 to 4%, and most preferably, from 1 to 2% (or 1.45 to 1.65%) by weight C18:2, and 5 to 22%, and preferably, 6.5 to 20%, and most preferably, from 7.5 to 18% (or 8.5 to 17.5%) by weight in total of C₈ and C₁₀ carbon chains based on total weight of hydrophobic tail on the zwitterionic surfactant, wherein the gum is selected from the group consisting of hydroxypropyltrimethyl ammonium chloride guar, locust bean gum, konjac mannan gum, gum tragacanth, sodium or propylene glycol alginate, kappa-, iota-, or lambda-carrageenan, guar, hydroxylpropyl guar gum, karaya gum, gum arabic, gellan, xanthan, succinoglycan or its acidic or enzymatic hydrolysates, agar, pectin, and mixtures thereof.

The present disclosure also encompasses a method for washing a surface, including skin on the face and/or body, by contacting the surface with at least one of the wash compositions of the first three aspects of the invention.

All other aspects of the present invention will more readily become apparent from the description and examples which follow.

Skin, as used herein, is meant to include skin on the arms (including underarms), face, feet, neck, chest, hands, legs, buttocks and scalp (including hair). Cellulose includes a water insoluble polysaccharide composed of chains of glucose molecules linked via *beta* 1-4 glycosidic bonds or linkages. Cellulose also includes water soluble chemically or physically modified polysaccharides and polysaccharides dispersible in water when used in combination with water soluble cellulose. Starch, as used herein, means a water-soluble polysaccharide made up of glucose molecules that are joined by linkages that include *alpha* 1-4 and/or 1-6 glycosidic bonds or linkages. Gum as used herein means a thickening agent which can be, for example, a polysaccharide produced by the fermentation of carbohydrates using a gram-negative bacterium (i.e., *Xanthomonas campestris*), a soluble fiber having a protein content typically ranging from 5 to 6% obtained from the endosperm of guar seeds or the like. Unsaturated zwitterionic surfactant means a surfactant like a betaine having at least one double bond (a CₙH₂ₙ bond), and preferably, one double bond in the surfactant hydrophobic (i.e., fatty) chain. As used herein, C18:1 and C18:2 means an 18-carbon chain with 1 and 2 double bonds in the chain, respectively. Wash composition means a composition suitable for use on a surface, including a hard surface such as a table, counter, appliance, window, ceramic fixture (like a toilet or sink), automobile and floor. Surface also includes skin, and preferably, is skin. The wash composition of the present invention, therefore, is suitable to be a home care composition, shampoo, make-up remover, facial wash or personal care and liquid body or hand wash. Preferably, the wash composition of the present invention is a shampoo, body wash or hand wash that is ready for topical application and to be wiped or washed off, and preferably, washed off with water. The wash composition may, optionally, comprise medicinal or therapeutic agents, but preferably, is a wash which is a cleaning and/or cosmetic wash that is a non-therapeutic wash to wash of, for example, dirt, oils, and/or bacteria that can cause staining and/or malodour. As hereinafter described, the wash composition of the present invention may optionally comprise skin benefit ingredients added thereto such as vitamins and/or derivatives/analogues thereof, resorcinols, retinoic acid precursors, colorants, moisturizers, sunscreens, mixtures thereof or the like. The skin benefit ingredients (or agents) may be water or oil soluble. If used, oil soluble skin benefit agents typically make up to 1.5% by weight of the wash composition whereby water-soluble skin benefit agents, when used, can make up to 10% by weight of the wash composition. The wash composition typically has a pH from 5.25 to 7.75, and preferably, 5.8 to 7.5, and most preferably, 6.2 to 7.4 (or 6.55 to 7.35). Viscosity, unless noted otherwise, is taken with a Discovery HR-2 Rheometer using sand blasted plates having a 1000 micron gap and a first shear rate S_{A} of 4 _{S}⁻¹ for a first viscosity V_{A} and a second shear rate S_{B} of 10 s⁻¹ for a second viscosity V_{B}, both at 25°C and 20 second intervals. Viscosity is reported in millipascal seconds (1 millipascal seconds = 1 centipoise (cps)). Stable, as used herein, means no phase separation, discoloration and malodour generation from the wash composition after being stored for at least two (2) weeks at 50°C, and preferably, at least one (1) month at 45°C, and more preferably, from 2 to 4 months at 45 °C. Rinses easily means washes off with water and without leaving a sticky or tacky residue feeling as determined by trained panelists. Biodegradable, as used herein, means the breakdown of organic matter like gum, starch and cellulose by microorganisms, such as bacteria and fungi. Substantially free of means less than 3.5%, and preferably, less than 2.5%, more preferably, less than 1.25%, and most preferably, less than 0.65% by weight of the of the wash composition. Substantially free of is meant to include from 0.001 to less than 3.5%, and also, 0.0% (i.e., no or none) by weight of the composition. Predominately free of, as used herein, means less than 1%, and preferably, less than 0.35%, and most preferably, less than 0.25% (or less than 0.15% or less than 0.05%). In an embodiment of the invention, predominately free of includes 0.001 to 1.0% and 0.0% (i.e., no or none) by weight of the wash composition. The term comprising is meant to encompass the terms consisting essentially of and consisting of. For the avoidance of doubt, and for illustration, the composition of this invention comprising surfactant and biodegradable thickener is meant to include a composition consisting essentially of the same and a composition consisting of the same. Except in the operating comparative examples, or where otherwise explicitly indicated, all numbers in this description indicating amounts or ratios of materials or conditions and/or physical properties of materials and/or use are to be understood as modified by the word "about". All ranges defined herein are meant to include all ranges subsumed therein unless otherwise stated.

### Detailed Description of the Invention

The thickener is preferably biodegradable thickener. Accordingly, the thickener preferably consists of organic matter. As to the biodegradable thickener suitable for use, the same comprises starch, and cellulose and/or gum where the thickener is not required to be synthetically manufactured and can completely decompose or disintegrate in the environment. In an embodiment of the invention, at least 90% by weight of the biodegradable thickener is starch, and cellulose and/or gum. In another embodiment, at least 92% by weight of the biodegradable thickener is starch, and cellulose and/or gum. In even another embodiment, at least 92 to 100% by weight of the biodegradable thickener is starch, and cellulose and/or gum. In still another embodiment, from 90 to 97% or 92 to 96% or 100% by weight of the biodegradable thickener is starch, and cellulose and/or gum.

Typically, the wash composition of the present invention will comprise from 1.0 to 10.5% by weight of the thickener, and preferably, from 3.5 to 10%, and most preferably, from 3.6 to 9.0% (or 3.65 to 7% or 3.7 to 6.65%) by weight of the thickener. In another embodiment of the invention, starch makes from 58 to 99% or from 65 to 98.5% or from 70 to 98.5% (or 77 to 98.5 or 82 to 98% by weight of the total weight of starch, and cellulose and/or gum in the biodegradable thickener. In an embodiment of the invention, 100% of the biodegradable thickener is starch, and cellulose and/or gum where cellulose makes up from 70 to 99% by weight of the total weight of the thickener.

In a further embodiment, 100% by weight of the biodegradable thickener is starch, and cellulose and/or gum whereby the same makes up from 2.75 to 7.75%, and preferably, from 3.0 to 6.85%, and most preferably, from 3.5 to 6.25% (or 3.05 to 4.45% or 3.1 to 3.75%) by weight of the wash composition. In an embodiment of the invention, 1 to 5 or 1 to 4 or 2 to 3 times more cellulose is used than gum when both are used in the wash composition of the invention.

Salt or electrolyte, like Na, Mg, Ca and/or ammonium chloride make(s) up from 0 to 2.5%, and preferably, from 0.1 to 1.35%, and most preferably, 0.5 to 1% by weight of the wash composition. In another embodiment, the wash composition comprises less than 0.5% or less than 0.25% by weight or no (0.0% by weight) salt or electrolyte.

As to the biodegradable thickener suitable for use, the starches include those that may optionally be chemically or physically modified by one or more art recognized techniques like oxidations, crosslinking reactions, gelatinizations, esterification, etherification, amidation and treatments with temperature variations. Distarch phosphates or compounds rich in distarch phosphate are starches often included for use in the present invention. Starch hydrosylates are also suitable for use.

Such suitable starches may originate or be derived from any plant source like corn, pea, potato, oat, rice, tapioca, sorghum, barley or wheat.

Often preferred for use are hydroxypropyl starch and distarch phosphates suitable for use, alone or in mixtures with other starches, include those made available from suppliers like Grain Processing Corp under the Pure-Gel^{®} name (e.g., B980, B990 food starch modified grade), Cargill Corporation under the StarDesign^{™} name (e.g., C☆EmTex^{®}) and Agrana under the Agenaflo name (e.g., 9050, 9051), Ingredion under the Farmal^{®} name (e.g., CS 3757) and Roquette^{®} (e.g., Zea Mays ST 005).

Other starch phosphates, and in particular hydroxypropyl starch phosphates, or compounds rich in starch phosphate suitable for use are sold by Avebe under the Prejel^{™} name. These include VA-70-T AGGL (gelatinized hydroxypropyl cassava distarch phosphate), TK1 (gelatinized cassava distarch phosphate) and 200 (gelatinized acetyl cassava distarch phosphate). These materials, also, may be used alone or in combination with other starches.

Starches suitable for use include starch and distarch phosphates like acetylated distarch phosphate, acetylated distarch adipate, starch sodium octenyl succinate, hydroxypropyl starch phosphate, hydroxypropyl distarch phosphate mixtures thereof or the like. Illustrative examples of starches suitable for use either alone or in mixtures include: StarDesign^{™} Care, StarDesign^{™} Care AV, StarDesign^{™} 05340, CAEmTex^{®} 06328, PolarTex^{®}, C☆PolarTex^{®}, C☆HiForm^{™}, C☆HiForm A^{™}, HiForm Starch^{™}, StarDesign^{™}, Pure-Gel^{®} B980, Pure-Gel^{®} B990, Pure-Gel^{®} B992, Pure-Gel^{®} B994, FARMAL^{®} AF 1100, FARMAL^{®} CS 3757, FARMAL MS 6822, FARMAL^{™} GMS 2143, FARMAL^{®} MS 6892, FARMAL^{®} MS 6135, FARMAL^{®} GMS 2141, FARMAL^{®} CS 21A, FARMAL^{®} CS 21R, FARMAL^{®} MS 6135, FARMAL^{®} GS 1653, FARMAL^{®} CS 3757, FARMAL^{®} GMS 2141, FARMAL^{®} CS 3001, FARMAL CS 3410, FARMAL^{®} CS 3400, FARMAL^{®} CS 3650, FARMAL^{®} WS 4400, FARMAL^{®} GMS 2141, Nativacare^{™} 8600, Nativacare^{™} 5600, Nativacare^{™} 9360, Nativacare^{™} 9230, Nativacare^{™} 9330; AGENAFLO 9050, AGENAFLO OS 9051, AGENAFLO TS, AGENAJEL 20.313, AGENAJEL 20.306, AGENAJEL 20.350, AGENAMALT 20.222, AGENANOVA 30.326, DEXTRIN 20.901, AGENABON 20.219, STRUCTURE^{®} XL, STRUCTURE^{®} 2143, STRUCTURE^{®} 6892, STRUCTURE^{®} STYLE or the like.

Hydroxypropyl distarch phosphate, sold as E1440 hydroxypropyl starch phosophate by Sinofi is also suitable for use.

As to the celluloses suitable for use as biodegradable thickeners, these include components generally classified as a carboxymethylcellulose, hydroxymethylcellulose, hydroxypropylcellulose, hydroxypropylmethyl cellulose, hydroxyethylcellulose or mixtures thereof.

Illustrative examples of such microcrystalline celluloses that may be used (alone or in a mixture with additional celluloses) or include Vivapur^{®} COS5, Vivapur^{®} COS6, Vivapur^{®} COS8, Vivapur^{®} CS 032 XV, Vivapur^{®} CS 152 HV, Vivapur^{®} CS 302 SV, Vivapur^{®} 12, Vivapur^{®} 14, Vivapur^{®} 101, Vivapur^{®} 102, Vivapur^{®} 103, Vivapur^{®} 105, Vivapur^{®} 112, Vivapur^{®} 200, Vivapur^{®} 301, Vivapur^{®} 302, Vivapur^{®} 200, Vivapur^{®} 200 XLM, Emcocel^{®} 50M, Emcocel^{®} 90M, Emcocel^{®} 90 XLM, Emcocel^{®} 50M, Emcocel^{®} 90M, Emcocel^{®} 90HD, Emcocel^{®} 200 LP made available from JRS Pharma GmbH & Co.

Others suitable for use (either alone or in a mixture with additional celluloses) include Avicel^{®} GP1412; Avicel^{®} 101, Avicel^{®} 102, Avicel^{®} 103, Avicel^{®} 112, Avicel^{®} 113, Avicel^{®} 301, Avicel^{®} 200, Avicel^{®} SMCC 50, Avicel^{®} SMCC 90, Avicel^{®} SMCC HD90, Avicel^{®} CL, Avicel^{®} DG, Avicel^{®} CE, Avicel^{®} HFE, Lattice^{®} NT-20, Lattice^{®} NTC-61, Lattice^{®} NT-50, Lattice^{®} NT-80, Lattice^{®} NT-100, Lattice^{®} NT-200, GRINDSTED^{®} Cellulose gum BAK, GRINDSTED^{®} Cellulose gum BEV, GRINDSTED^{®} Cellulose gum MAS, GRINDSTED^{®} Cellulose gum AMD, GRINDSTED^{®} Cellulose gum NMD, GRINDSTED^{®} Cellulose gum FZD, Methocel^{™} 240, Methocel^{™} E5, Methocel^{™} F4M, Methocel^{™} K4M, Methocel^{™} K15M, Methocel^{™} K15LV, Methocel^{™} F50, Methocel^{™} K100M, Methocel^{™} K 200M, Methocel^{™} 40-0100, Methocel^{™} 40-0202 and Methocel^{™} F50 and available from Dupont.

Comprecel^{®} 101, Comprecel^{®} 102, Comprecel^{®} 301, Comprecel^{®} 302 available from Ming Thai Chemical Co.; Microcel^{®} 101, Microcel^{®} 102, Microcel^{®} 200 available from Blanver, Farmoquimica; Farmal^{®} CMC 2700 F available from Ingredion; Arbalon^{®} R49, Arbalon^{®} R50 available from Lubrizol; Natrava^{®} Citrus Fibers, Arbalon^{®} Cellulose Liquid available from CP Kelco are also suitable for use. Even other celluloses that can be used (alone or as mixtures) include Exilva^{®} FM 02-V available from Borregaard; CelluForce NCC^{®} Cellulose NanoCrystals (CNC) available from CelluForce; CELOVA^{®} Celova Microfibrillated Cellulose (MFC) available from Weidmann Fiber Technology; FiberDesign^{™} Sensation available from Cargill, Natrava^{®} Citrus Fiber available from by CPKelco; CELLOSIZE^{™} QP-10000H EUR, CELLOSIZE^{™} QP-100MH, CELLOSIZE^{™} QP-300, CELLOSIZE^{™} QP-4400H, EUR CELLOSIZE^{™} QP-15000H, CELLOSIZE^{™} QP-30000H, CELLOSIZE^{™} QP-52000H, CELLOSIZE^{™} EP-09 , Methocel^{™} F50, Methocel^{™} 40-0100, Methocel^{™} 40-0202, Methocel^{™} K100M, Methocel^{™} K200M made available by Dow Chemical; Polysurf^{™} 67 CS, Natrosol^{™} Plus 330 CS, Natrosol^{™} Plus 250HHR CS available from Ashland; Methyl Cellulose PMK-40YS and PMK-60YS available by First Continental International Inc; Carboxymethyl Cellulose (CMC), Poly Anionic Cellulose (PAC), Hydroxyethyl Cellulose (HEC), Hydroxypropyl Methylcellulose (HPMC), and Methyl Hydroxyethyl Cellulose (MHEC/HEMC) made available by Kingsun Chemicals Inc.; SidleyChem^{®} Hydroxypropyl MethylCellulose (HPMC), Methyl Cellulose (MC), Sodium CarboxymethylCellulose (CMC), High Substitute Hydroxypropyl Cellulose (H-HPC), Low Substitute Hydroxypropyl Cellulose (L-HPC), Ethyl Cellulose(EC), Hydroxyethyl Cellulose (HEC), Microcrystalline Cellulose (MCC), Polyanionic Cellulose (PAC), Hydroxypropyl Methyl Cellulose Phthalate (HPMC-P)/(Hypromellose phthalate), Hydroxypropyl Methyl Cellulose Acetate Succinate (HPMCAS), Croscarmellose Sodium, Powdered Cellulose available from SIDLEY CHEMICAL CO., LTD.

The preferred celluloses (microcrystalline celluloses) used in the present invention are those made commercially available form Dupont and JRS Pharma GGmbH & Co.

In an embodiment of the invention, the gums used will include hydroxypropyltrimethyl ammonium chloride guar, locust bean gum, konjac mannan gum, gum tragacanth, sodium or propylene glycol alginate, kappa-, iota-, or lambda-carrageenan, guar, hydroxylpropyl guar gum, karaya gum, gum arabic (acacia), gellan, xanthan, succinoglycan or its acidic or enzymatic hydrolysates, agar, pectin, and mixtures thereof.

In an especially preferred embodiment, the gum selected for use is Jaguar^{®} S from Solvay, TICorganic^{®} Guar Gum 3500 Powder from Ingredion, Viscogum^{™} Guar Gum made commercially available from Cargill and/or Gellan Gum (anionic polysaccharide produced by the bacterium *Shingomonas elodea*) made commercially available from CP Kelco.

The biodegradable thickeners preferred typically have a bulk density from 0.18 to 0.92, and preferably, from 0.2 to 0.82, and most preferably, from 0.22 to 0.72 g/ml (or 1000kg/m³) where bulk density is the ratio of the mass of an untapped powder sample and its volume including the contribution of the interparticulate void volume (i.e., dependent on both the density of powder particles and the spatial arrangement of particles in a powder bed). Such thickeners typically will have a cloud point from 60 to 70°C and a weight average molecular weight from 55,000 to 1.5 million Daltons, and preferably, from 80,000 to 1.25 million Daltons, and most preferably, from 100,000 to 1.0 million Daltons.

Regarding the surfactants that may be used, the same are limited only to the extent that they are suitable for use in compositions that are topically applied to a surface, preferably skin. Again, the wash composition of the invention can include soaps and sulfates but it is substantially free of the same.

Anionic surfactants suitable for use in the wash composition of the present invention include aliphatic sulfonates, such as a primary alkane (e.g., C₈-C₂₂) sulfonate, primary alkane (e.g., C₈-C₂₂) disulfonate, C₈-C₂₂ alkene sulfonate, C₈-C₂₂ hydroxyalkane sulfonate or alkyl glyceryl ether sulfonate (AGS); or aromatic sulfonates such as alkyl benzene sulfonate. The anionic may also be an alkyl sulfate (e.g., C₁₂-C₁₈ alkyl sulfate) or alkyl ether sulfate (including alkyl glyceryl ether sulfates). Among the alkyl ether sulfates are those having the formula:

RO(CH₂CH₂O)ₙSO₃M

wherein R is an alkyl or alkenyl having 8 to 18 carbons, preferably 12 to 18 carbons, n has an average value of at least 1.0, preferably less than 5, and most preferably 1 to 4, and M is a solubilizing cation such as sodium, potassium, ammonium or substituted ammonium.

The anionic may also include alkyl sulfosuccinates (including mono- and dialkyl, e.g., C₈-C₂₂ sulfosuccinates); acyl taurates (often methyl taurates or sodium lauroyl methyl taurate), acyl sarcosinates, sulfoacetates, C₈-C₂₂ alkyl phosphates and phosphonates, alkyl phosphate esters and alkoxyl alkyl phosphate esters, acyl lactates, C₈-C₂₂ monoalkyl succinates and maleates, alkyl glucosides and acyl isethionates, or the like.

Sulfosuccinates may be monoalkyl sulfosuccinates having the formula:

R¹O₂CCH₂CH(SO₃M)CO₂M;

and amide-MEA sulfosuccinates of the formula:
R¹CONHCH₂CH₂O₂CCH₂CH(SO₃M)CO₂M wherein R¹ ranges from C₈-C₂₂ alkyl.

Sarcosinates are generally indicated by the formula:
R²CON(CH₃)CH₂CO₂M, wherein R² ranges from C₈-C₂₀ alkyl.

Taurates are generally identified by formula:

R³CON R⁴CH₂CH₂SO₃M

wherein R³ is a C₈-C₂₀ alkyl, R⁴ is a C₁-C₄ alkyl.

M is a solubilizing cation as previously described. The isethionates that may be used include C₈-C₁₈ acyl isethionates (including those which have a substituted head group such as a C₁₋₄ alkyl substitution, preferably methyl substitution). These esters are prepared by a reaction between alkali metal isethionate with mixed aliphatic fatty acids having from 6 to 18 carbon atoms and an iodine value of less than 20. Often at least 75% of the mixed fatty acids have from 12 to 18 carbon atoms and up to 25% have from 6 to 10 carbon atoms.

Acyl isethionate suitable for use (i.e., anionic) may be an alkoxylated isethionate such as is described in Ilardi et al., U.S. Pat. No. 5,393,466, entitled "Fatty Acid Esters of Polyalkoxylated isethonic acid; issued Feb. 28, 1995; hereby incorporated by reference. This compound has the general formula:

R⁵C-O(O)-C(X)H-C(Y)H-(OCH₂-CH₂)ₘ-SO₃M

wherein R⁵ is an alkyl group having 8 to 18 carbons, m is an integer from 1 to 4, X and Y are each independently hydrogen or an alkyl group having 1 to 4 carbons and M is a solubilizing cation as previously described.

In an embodiment of the invention, the anionic surfactant suitable and preferred for use is sodium lauroyl isethionate, sodium cocoyl isethionate, sodium methyl lauroyl taurate, sodium methyl cocoyl taurate or a mixture thereof. Such anionic surfactants are commercially available from suppliers like Galaxy Surfactants, Clariant, Sino Lion and Innospec.

The wash composition can also comprise glutamates and glycinates as anionic surfactants either alone or in combination with other anionic surfactants, especially taurates and/or isethionates. If used, illustrative examples suitable for use include C₆ to C₁₈ glycinates and glutamates including sodium lauroyl glycinate, sodium cocoyl glycinate, sodium lauroyl glutamate, sodium cocoyl glutamate or mixtures thereof.

Amphoteric surfactants suitable for use in the invention (which depending on pH can be zwitterionic) include sodium acyl amphoacetates, sodium acyl amphopropionates, disodium acyl amphodiacetates and disodium acyl amphodipropionates where the acyl (i.e., alkanoyl group) can comprise a C₇-C₁₈ alkyl portion. Illustrative examples of the amphoteric surfactants suitable for use include sodium lauroamphoacetate, sodium cocoamphoacetate, sodium lauroamphoacetate, sodium cocoamphoacetate or mixtures thereof.

As to the zwitterionic surfactants that may be employed in the wash compositions of the present invention, such surfactants include at least one acid group. Such an acid group may be a carboxylic or a sulphonic acid group. They often include quaternary nitrogen, and therefore, can be quaternary amino acids. They should generally include alkyl and alkenyl or alkenyl groups of 7 to 18 carbon atoms and generally comply with an overall structural formula:

R⁶-[-C(O)-NH(CH₂)_{q}-]ᵣ-W-(R⁷-)(R⁸)A-B

where R⁶ is alkyl and alkenyl or alkenyl groups of 6 to 18 carbon atoms; R⁷ and R⁸ are each independently alkyl, hydroxyalkyl or carboxyalkyl of 1 to 3 carbon atoms; q is 2 to 4; r is 0 to 1; A is alkylene of 1 to 3 carbon atoms optionally substituted with hydroxyl, and B is --CO₂-- or-SO₃--.

Suitable zwitterionic surfactants that may be used in the present invention and within the above general formula include simple betaines of formula:

R⁶-N+-(R⁷)(R⁸)CH₂CO₂⁻

and amido betaines of formula:
R⁶-CONH(CH₂)ₜ-N⁺-(R⁷)(R⁸)CH₂CO₂⁻where t is 2 or 3.

In both formulae R⁶, R⁷ and R⁸ are as defined previously. R⁶ may, in particular, include a mixture of C₈, C₁₀, C₁₂ and C₁₄ alkyl groups derived from coconut oil. R⁷ and R⁸ are preferably methyl. R⁶ will preferably include a mixture of C₁₂ and C₁₄ alkenyl groups preferably derived from triglycerides. Triglycerides recovered from asparagus, corn, canola or the like are suitable for use and sustainable.

A further possibility is that the zwitterionic surfactant comprises a sulphobetaine of formula:

R⁶--N⁺--(R⁷)(R⁸)(CH₂)₃SO₃⁻

or

R⁶-CONH(CH₂)ᵤ -N⁺-(R⁷)(R⁸)(CH₂)₃SO₃

where u is 2 or 3, or variants of these in which --(CH₂)₃SO₃⁻ is replaced by-CH₂C(OH)(H)CH₂SO₃⁻.

In these formulae, R⁶, R⁷ and R³ are as previously defined.

Illustrative examples of the zwitterionic surfactants suitable for use include betaines like oleoamidopropyl betaine, linoleoamidopropyl betaine, cocodimethyl carboxymethyl betaine, cocamidopropyl betaine and laurylamidopropyl betaine. An additional zwitterionic surfactant suitable for use includes cocamidopropyl hydroxy sultaine. Such surfactants are made commercially available from suppliers like Stepan Company, and it is within the scope of the invention to employ mixtures of the aforementioned surfactants. In a preferred embodiment, the zwitterionic surfactant used in the wash composition of this invention is a mixture of oleoamidopropyl betaine and linoleoamidopropyl or betaine cocamidopropyl betaine, oleoamidopropyl betaine and linoleoamidopropyl betaine.

Typically, total surfactant used in the wash composition of the invention is from 2.5 to 15%, and preferably, from 2.5 to 13%, and most preferably, from 2.5 to 12% (or 3.5 to 12%) by weight of the composition. Often, total anionic surfactant makes up from 0.75 to 8%, and preferably, from 2 to 7%, and most preferably, from 2.5 to 6% (or 2.55 to 5.85%) by weight of the composition. Zwitterionic surfactant makes up from 1 to 9%, and preferably, from 2.25 to 8%, and most preferably, from 2.4 to 7.5% (or from 2.5 to 6.15%) by weight of the was composition.

Nonionic surfactants may be used in the wash composition of the present invention. When used, nonionic surfactants are typically used at levels as low as 0.01, 0.5, 1, 1.5, or 2% by weight of the wash composition. In an embodiment of the invention, when nonionics are used, they make up from 0.01 to 0.485% by weight of the wash composition. The nonionics which may be used include, in particular, the reaction products of compounds having a hydrophobic group and a reactive hydrogen atom, for example aliphatic alcohols, acids, amides or alkylphenols with alkylene oxides, especially ethylene oxide either alone or with propylene oxide. Specific nonionic surfactant compounds are alkyl (C₆-C₂₂) phenols ethylene oxide condensates, the condensation products of aliphatic (C₈-C₁₈) primary or secondary linear or branched alcohols with ethylene oxide, and products made by condensation of ethylene oxide with the reaction products of propylene oxide and ethylenediamine. Other nonionic surfactants include long chain tertiary amine oxides, long chain tertiary phosphine oxides, dialkyl sulphoxides, and the like. Cocamide monoethanolamine (MEA) is an often a preferred optional nonionic surfactant suitable for use. When desired for use, MEA is used at levels from 0.2 to 0.46% by weight of the wash composition.

In an embodiment of the invention, nonionic surfactants optionally used can include fatty acid/alcohol ethoxylates having the following structures a) HOCH₂(CH₂)ₛ(CH₂CH₂O), H or b) HOOC(CH₂)_{c}(CH₂CH₂O)_{d} H; where s and v are each independently an integer up to18; and c and d are each independently an integer from 1 or greater. In an embodiment of the invention, s and v are each independently 6 to 18; c and d are each independently 1 to 30. Other options for nonionic surfactants include those having the formula HOOC(CH₂)ᵢ-CH=CH-(CH₂)ₖ(CH₂CH₂O), H, where i, k are each independently 5 to 15; and z is 5 to 50. In another embodiment of the invention, i and k are each independently 6 to 12; and z is 15 to 35.

The nonionic may also include a sugar amide, such as a polysaccharide amide. Specifically, the surfactant may be one of the lactobionamides described in U.S. Pat. No. 5,389,279 to Au et al., entitled "Compositions Comprising Nonionic Glycolipid Surfactants issued Feb. 14, 1995; which is hereby incorporated by reference or it may be one of the sugar amides described in U.S. Pat. No. 5,009,814 to Kelkenberg, titled "Use of N-Poly Hydroxyalkyl Fatty Acid Amides as Thickening Agents for Liquid Aqueous Surfactant Systems" issued Apr. 23, 1991; hereby incorporated into the subject application by reference.

In an embodiment of the invention, cationic surfactants may optionally be used in the wash composition of the present invention.

One class of optional cationic surfactants includes heterocyclic ammonium salts such as cetyl or stearyl pyridinium chloride, alkyl amidoethyl pyrrylinodium methyl sulfate, and lapyrium chloride.

Tetra alkyl ammonium salts are another useful class of cationic surfactants suitable for optional use. Examples include cetyl or stearyl trimethyl ammonium chloride or bromide; hydrogenated palm or tallow trimethylammonium halides; behenyl trimethyl ammonium halides or methyl sulfates; decyl isononyl dimethyl ammonium halides; ditallow (or distearyl) dimethyl ammonium halides, and behenyl dimethyl ammonium chloride.

Still other types of cationic surfactants that may be used are the various ethoxylated quaternary amines and ester quats. Examples include PEG-5 stearyl ammonium lactate (e.g., Genamin KSL manufactured by Clariant), PEG-2 coco ammonium chloride, PEG-15 hydrogenated tallow ammonium chloride, PEG 15 stearyl ammonium chloride, dipalmitoyl ethyl methyl ammonium chloride, dipalmitoyl hydroxyethyl methyl sulfate, and strearyl amidopropyl dimethylamine lactate.

Even other useful cationic surfactants suitable for optional use include quaternized hydrolysates of silk, wheat, and keratin proteins, and it is within the scope of the invention to use mixtures of the aforementioned cationic surfactants.

If used, cationic surfactants will make up no more than 1.0% by weight of the wash composition. If present, they typically make up from 0.01 to 0.7%, and more typically, from 0.1 to 0.5% by weight of the wash composition, including all ranges subsumed therein. In an embodiment of the invention less than 0.2% by weight cationic surfactant is used. In another embodiment, 0.0% by weight cationic surfactant is used.

In an embodiment of this invention, the wash composition will comprise less than 0.18% polymeric quaternary ammonium compounds (including salts of the same). In another embodiment, the wash composition will comprise less than 0.1% by weight polymeric quaternary ammonium compounds. In yet another embodiment, the wash composition comprises less than 0.01% by weight polymeric quaternary ammonium compounds. In even another embodiment, the wash composition is free of polymeric quaternary ammonium compounds (i.e., 0.0%).

While not desired, fatty acid soaps like sodium stearate may be included in the composition but the wash compositions are preferably substantially free of soaps. In an embodiment of the invention, soaps will make up less than 1.0% by weight of the wash composition, and preferably, less than 0.5% (or 0.3%), and most preferably, 0.0% by weight of the wash composition of the present invention.

The wash composition of the present invention is again as herein defined substantially free of sulfates, and/or synthetic thickeners like ammonium acryloyldimethyltaurate vinylpyrrolidone copolymer, alkyl acrylate crosspolymers or the like.

Water typically makes up from 59 to 95%, and preferably, from 65 to 90%, and most preferably, from 65 to 80% by weight of the wash composition.

Adjusters suitable to modify/buffer the pH may optionally be included. Such pH adjusters include triethylamine, NaOH, KOH, H₂SO₄, HCl, C₆H₈O₇ (i.e., citric acid) or mixtures thereof. The pH adjusters are added at amounts to yield the desired final pH. The pH values may be assessed with commercial instrumentation such as a pH meter made commercially available from Thermo Scientific^{®} or VWR Scientific. Such pH values of the wash composition are as noted typically from 5.25 to 7.75, and preferably, 5.8 to 7.5, and most preferably, 6.2 to 7.4 (or 6.55 to 7.35).

Polyols suitable for use in the wash composition are limited only to the extent that they are usable in a wash composition for topical application. Illustrative and non-limiting examples of the polyols suitable for use in the present invention include sorbitol, glycerol, mannitol, xylitol, maltitol or mixtures thereof. In an embodiment of the invention, the polyol used is typically at least 50% by weight glycerol, based on total weight of the polyol used in the wash composition. In another embodiment of the invention, the polyol used is all glycerol (100% by weight). Polyol will typically make up from 0.0 to 25% by weight of the wash composition, and preferably, from 0.5 to 9% by weight of the wash composition, and most preferably, from 0.75 to 5.5% (or 0.95 to 3.65%) by weight of the wash composition.

While not required, in addition to the mixture of thickener having starch, cellulose and/or gum of the present invention, it is within the scope of this invention to optionally use additional gelling or thickening agents like ammonium acryloyldimethyltaurate vinylpyrrolidone copolymer, alkyl acrylate crosspolymers or the like. If used, the acrylates include those generally classified as acrylic acid crosslinked with allyl sucrose or allyl pentaerythritol acrylic acid and C₁₀-C₃₀ alkyl acrylate crosslinked with allyl pentaerythritol. Such acrylates are sold under the Carbopol^{®} and Ultrez^{®} names and are made commercially available by Lubrizol. Other acrylates suitable for use include those which are a copolymer formed from an ester of acrylic acid and ethoxylated palm alcohol with about 25 moles of ethylene oxide and one or more monomers of acrylic acid, methacrylic acid or one of their simple esters. These acrylates are sold, for example, under the Synthalen^{®} (i.e., W200, W2000) names, and made commercially available from suppliers like 3V Sigma USA. As noted, the wash compositions are predominately free of such optional thickeners and the same will make up less than 1% by weight of the wash composition. Again, and even more preferably, no (0.0%) thickener in addition to starch, cellulose and/or gum is used in the wash compositions.

The viscosity of the wash composition (measured at 4 s⁻¹ for 20 seconds) is typically 40,000 cps or less, and preferably, from 700 to 15,000 cps, and most preferably, from 2000 to 13,500 cps.

Optional skin benefit agents suitable for use in the wash composition are limited only to the extent that they are capable of being topically applied, and suitable to remain stable in the wash composition at the desired pH.

Illustrative examples of the benefit agents suitable to include in the water portion of the wash composition are acids, like amino acids, such as arginine, valine or histidine. Additional water soluble benefit agents suitable for use include vitamin B₂, niacinamide (vitamin B₃), vitamin B5 (pantothenic acid), vitamin B₆, vitamin C, mixtures thereof or the like. Water soluble derivatives of such vitamins may also be employed. For instance, vitamin C derivatives such as ascorbyl tetraisopalmitate, magnesium ascorbyl phosphate and ascorbyl glycoside may be used alone or in combination with each other. Other water-soluble benefit agents suitable for use include 4-ethyl resorcinol, extracts like sage, aloe vera, green tea, grapeseed, thyme, chamomile, yarrow, cucumber, liquorice, rosemary extract or mixtures thereof. Water soluble sunscreens like ensulizole may also be used. Total amount of optional water-soluble benefit agents (including mixtures) when present in the wash composition may range from 0.0 to 10%, preferably from 0.001 to 8%, and most preferably, from 0.01 to 6% (or 0.001 to 2% or 0.001 to 1%) by weight of the wash composition.

It is also within the scope of the present invention to optionally include oil soluble benefit agents. Such oil soluble actives or benefit agents can be solubilized in the surfactants used. The only limitation with respect to such oil soluble benefit agents are that the same are suitable to provide a benefit when topically applied.

Illustrative examples of the types of oil soluble benefit agents that may optionally be used in the compositions of this invention include components like stearic acid, vitamins like Vitamin A, D, E and K (and their oil soluble derivatives), sunscreens like ethylhexylmethoxycinnamate, bis-ethyl hexyloxyphenol methoxyphenol triazine, 2-ethylhexyl-2-cyano-3,3-diphenyl-2-propanoic acid, drometrizole trisiloxane, 3,3,5-trimethyl cyclohexyl 2-hydroxybenzoate, 2-ethylhexyl-2-hydroxybenzoate or mixtures thereof.

Other optional oil soluble benefit agents suitable for use include resorcinols like 4-hexyl resorcinol, 4-phenylethyl resorcinol, 4-cyclopentyl resorcinol, 4-cyclohexyl resorcinol 4-isopropyl resorcinol, thiamidol (Isobutylamido thiazolyl resorcinol) or a mixture thereof. Also, 5-substituted resorcinols like 4-cyclohexyl-5-methylbenzene-1,3-diol, 4-isopropyl-5-methylbenzene-1,3-diol, mixtures thereof or the like may be used. The 5-substituted resorcinols, and their synthesis are described in commonly assigned U.S. Published Patent Application No. 2016/0000669A1.

Even other oil soluble actives suitable for use include omega-3 fatty acids, omega-6 fatty acids, climbazole, farnesol, ursolic acid, myristic acid, geranyl geraniol, oleyl betaine, cocoyl hydroxyethyl imidazoline, hexanoyl sphingosine, 12-hydroxystearic acid, petroselinic acid, conjugated linoleic acid, terpineol, thymol mixtures thereof or the like.

When optional oil soluble active or benefit agent is used in the composition, such active typically makes up from 0.0 to 1.5%, and preferably, from 0.001 to 0.65%, and most preferably, from 0.05 to 0.35% by weight of the wash composition. In yet another embodiment, oil soluble benefit agent makes up from 0.1 to 0.5% by weight of the total weight of the wash composition.

In an embodiment of the invention, the optional oil soluble benefit agent used is a retinoic acid precursor. In one embodiment of the invention, the retinoic acid precursor is retinol, retinal, retinyl propionate, retinyl palmitate, retinyl acetate or a mixture thereof. Retinyl propionate, retinyl palmitate and mixtures thereof are typically preferred. In yet another embodiment, from 0.01 to 1%, or from 0.01 to 0.65% by weight 12-hydroxystearic acid is used in the wash composition of the invention.

Other optional additives suitable for use include zinc pyrithione, octopirox and mixtures thereof. When used, they typically are employed from 0.001 to 1.5%, and preferably, from 0.01 to 1.25% by weight of the wash composition.

Conventional preservatives can desirably be incorporated into the wash composition to protect against the growth of potentially harmful microorganisms. Cosmetic chemists are familiar with appropriate preservatives and routinely choose them to satisfy the preservative challenge test and to provide product stability. Suitable traditional preservatives for use include hydantoin derivatives and propionate salts. Particularly preferred preservatives are iodopropynyl butyl carbamate, phenoxyethanol, hydroxyacetophenone, ethylhexylglycerine, hexylene glycol, methyl paraben, propyl paraben, imidazolidinyl urea, sodium dehydroacetate, dimethyl-dimethyl (DMDM) hydantoin, sodium benzoate, benzyl alcohol and mixtures thereof. Other preservatives suitable for use include sodium dehydroacetate, chlorophenesin, decylene glycol, N-capryloyl glycine, N-undecylenoyl glycine and mixtures of the same. The preservatives should be selected having regard for the use of the composition and possible incompatibilities between the preservatives and other ingredients in the composition. Preservatives are preferably employed in amounts ranging from 0.01% to 2.0% by weight of the total weight of the wash composition. A preservative system that includes from 0.1 to 1.0% 1,2-octane diol based on total weight of the wash composition is suitable for use. Preservative systems substantially free of parabens and hydantoins are preferred. Sodium benzoate is an often most preferred preservative and employed typically at from 0.2 to 1.2% by weight of the wash composition.

Fragrances, fixatives, chelators (like EDTA), opacifiers (like titanium dioxide and vegetable-derived liquid dispersions like MACKADET^{®} OPR 2 made available from Solvay), inorganics, dyes, pigments and exfoliants may optionally be included in the wash composition. Each of these substances may range from about 0.03 to about 5%, preferably between 0.1 and 3%, and most preferably, from 0.1 to 2% by weight of weight of the wash composition. To the extent the exfoliants are used, those selected should be of small enough particle size so that they do not impede the performance of any packaging used to dispense the compositions of this invention. Fragrances often make up from 0.0 to 1.5%, and preferably, from 0.2 to 1% by weight of the wash composition.

Conventional emulsifiers having an HLB of greater than 8 may optionally be used. Illustrative examples include Tween, 40, 60, 80, polysorbate 20 and mixtures thereof. Such emulsifiers, when used for water continuous systems, make up from 0.03 to 1.5% by weight of the wash composition.

Regarding silicones like dimethicone or the like, the wash compositions will typically comprise less than 1.0% by weight silicone, more preferably, less than 0.5% by weight silicone, and most preferably, no silicone. In an especially preferred embodiment, the wash composition of the present invention is free of hydantoins, parabens, sulfates and silicones, especially cyclic silicones like decamethylcyclopentasiloxane.

The wash compositions of the present invention is preferably a liquid wash composition suitable to be in an isotropic or lamellar phase.

When lamellar, the composition of the invention utilizes from 0.1 to 20%, and preferably, from 1 to 10%, and most preferably from 2 to 5% by weight of a lamellar structuring agent (based on total weight of liquid wash composition) which works in the compositions to form the lamellar phase.

Examples of fatty acids (or ester derivative, or fatty alcohol, or trihydroxystearin) which may be used as structurants are C₈-C₂₂ fatty acids like caprylic acid, lauric acid, myristic acid, oleic acid, isostearic acid, linoleic acid, linolenic acid, ricinoleic acid, elaidic acid, arachidonic acid, myristoleic acid and palmitoleic acid, mixtures thereof or the like. Ester derivatives include propylene glycol isostearate, propylene glycol oleate, glyceryl isostearate, glyceryl oleate and polyglyceryl diisostearate, or the like. The structurant is preferably a fatty acid. More preferably, the structurant is selected from the group consisting of caprylic acid, lauric acid, myristic acid or a mixture thereof.

When making wash composition of the present invention, the desired ingredients may be mixed with conventional apparatus under moderate shear and atmospheric conditions, with temperature ranging from 30 to 85°C whereby shear continues until a homogeneous product is recovered.

The packaging for the wash composition typically is not limited as long as composition can be dispensed. In an embodiment on the invention, the wash composition is sold in a pouch, bottle, jar, tube or canister. The packaging preferably allows for infinite numbers of refilling to invariably reduce plastic waste in the environment, and most preferably, has at least 50% by weight post-consumer resin. The wash compositions of the present invention may be sold in a concentrated form whereby the consumer can be instructed to add water to generate product ready for use and in an effort to reduce weight when shipping and over reliance on virgin plastic.

The Examples are provided to facilitate an understanding of the invention. They are not intended to limit the scope of the claims.

### Examples

All Samples in the Examples were prepared by mixing the ingredients with moderate shear. Temperature was varied from about 60 to 78°C and pressure while mixing was atmospheric. The starch, cellulose and gums used in the Samples are as identified in the Examples which follow: the starch used was StarDesign^{™} Care provided commercially from Cargill, the cellulose used was Vivapur^{®} COS6 provided commercially from JRS Pharma GmbH & Co., and the gums used were guar gum Jaguar^{®} S from Solvay, TICorganic^{®} Guar Gum 3500 Powder from Ingredion, Carrageenan Genuvisco^{®} CG-131 from CP Kelco, Gellan Gum Kelcogel^{®} CG-HA from CP Kelco.

The base wash compositions were made with the ingredients identified where starch, cellulose and gum were added to the base compositions in the amounts described in the Samples of Example II.

### Example I.

**TABLE I**

| Wash Composition Bases | | |
|---|---|---|
| Ingredients | Wash Base 1 (weight%) | Wash Base 2 (weight%) |
| Sodium methyl lauroyl taurate | | 2.1 |
| Sodium lauroyl glycinate | 3.24 | |
| Sodium lauroyl isethionate | 2.23 | 2.1 |
| Zwitterionic/Betaine* | 3.04 | 6.5 |
| Cocamide MEA | | |
| Lauric acid | 0.92 | 0.5 |
| Stearic acid | 0.31 | 0.31 |
| Glycerol | 0.88 | 1.0 |
| 12-hydroxystearic acid | | 0.05 |
| Glycerol monostearate | | 0.05 |
| Preservative | 0.85 | 0.85 |
| Opacifier | 1.0 | 1.0 |
| NaOH | 0.05 | 0.18 |
| pH adjuster | To target pH | To target pH |
| Biodegradable Thickener | As in Example 2 | As in Example 2 |
| Fragrance | 1.0 | 1.0 |
| Water and Minors | balance | balance |

pH of the wash compositions, 6.8 to 7.2

**TABLE II**

| *Carbon chain length distribution of zwitterionic surfactant: % by weight hydrophobic portion of zwitterionic surfactant (betaine) | | | | | | | | |
|---|---|---|---|---|---|---|---|---|
| Origin | C8 | C10 | C12 | C14 | C16 | C18 | C18:1 | C18:2 |
| Hydrophobic Chain unsaturated | 5-8 | 5-8 | 47-53 | 15-21 | 8-12 | 4 Max. | 5-12 | 2 Max. |
| Fatty acid saturated | 0.1 Max. | 0.1 Max. | 48-58 | 14-20 | 7-12 | 14-24 | 0.5 Max. | |

Max.-maximum amount in the hydrophobic portion of the zwitterionic surfactant based on total weight of the hydrophobic portion and greater than 0.0%

### Example II.

The biodegradable thickeners identified in TABLE III were used in combination with the wash compositions identified in Example I, Wash Base 2.

**Table III**

| Starch | Cellulose | Iota Carageenan | Gellan Gum | Guar Gum | Stability | |
|---|---|---|---|---|---|---|
| | | | | | Saturated Zwitterionic | *Unsaturated Zwitterionic |
| 4.3 | 0.75 | | | | Unstable | |
| 4.3 | 0.75 | | | | | Stable |
| 3.5 | | 0.22 | | 0.11 | Unstable | |
| 4.5 | | 0.22 | | 0.11 | | Stable |
| 2.0 | | | 0.15 | | Unstable | |
| 5.5 | | | 0.15 | | | Stable |

The wash compositions were stored at elevated temperatures, not exceeding 50°C. In less than one week, compositions not made according to the present invention (i.e., not comprising unsaturated zwitterionic as herein described) discolored and separated and were not usable wash compositions. In contrast, the wash compositions made according to the invention were stable, did not discolor, and maintained viscosity even after being stored at 45°C for 1 month. Moreover, skilled panelists concluded that the compositions made according to the invention provided a desirable sensory and washed off well when used.

After washing the hands and forearms with the compositions made consistent with the invention, all trained panelists concluded that the wash compositions rinsed off easily, left a filmless sensation and lathered well. Also, the panelists surprisingly concluded the compositions made according to the invention performed similar to those made with traditional polymeric synthetic thickeners.

### Example III

The biodegradable thickeners identified in TABLE IV were used in combination with wash compositions similar to those identified in Example I, except that the surfactant system used contained sodium lauroyl isethionate, sodium methyl lauryl taurate and zwitterionic as identified in a weight percent ratio of 2, 2, 7 respectively. Saturated betaine was cocamidopropyl betaine, i.e., C₁₂ saturated.

**Table IV**

| Starch | Cellulose | Iota Carageenan | Gellan Gum | Guar Gum | Stability | |
|---|---|---|---|---|---|---|
| | | | | | Saturated Zwitterionic | *Unsaturated Zwitterionic |
| 4.3 | 0.75 | | | | Unstable | |
| 4.3 | 0.75 | | | | | Stable |
| 3.5 | | 0.22 | | 0.11 | Unstable | |
| 4.5 | | 0.22 | | 0.11 | | Stable |
| 2.0 | | | 0.15 | | Unstable | |
| 5.5 | | | 0.15 | | | Stable |
| | | | | | | |

The wash compositions were stored at elevated temperatures, not exceeding 50°C. In less than one week, compositions not made according to the present invention (i.e., not comprising unsaturated zwitterionic as herein described) discolored and separated and were not usable wash compositions. In contrast, the wash compositions made according to the invention were stable, did not discolor, and maintained viscosity even after being stored at 45°C for 1 month.

After washing the hands and forearms with the compositions made consistent with the invention, all trained panelists concluded that the wash compositions rinsed off easily, left a filmless sensation and lathered well. Also, the panelists surprisingly concluded the compositions made according to the invention performed similar to those made with traditional polymeric synthetic thickeners.

### Example IV

The biodegradable thickeners identified in TABLE V were used in combination with the wash compositions identified in Example I, Wash Base I.

**Table V**

| Starch | Cellulose | Iota Carageenan | Stability | |
|---|---|---|---|---|
| | | | Saturated Zwitterionic | *Unsaturated Zwitterionic |
| 3.5 | | | Unstable | |
| | 0.75 | | Unstable | |
| 3.5 | 0.75 | | Unstable | |
| 3.5 | 0.75 | | | Stable |
| 3.5 | | 0.5 | Unstable | |
| 3.5 | | 0.5 | | Stable |
| 3.5 | | 0.75 | | Stable |
| 3.5 | | 0.65 | | Stable |

The wash compositions were stored at elevated temperatures, not exceeding 50°C. In less than one week, compositions not made according to the present invention (i.e., not comprising unsaturated zwitterionic as herein described) discolored and separated and were not usable wash compositions. In contrast, the wash compositions made according to the invention were stable, did not discolor, and maintained viscosity even after being stored at 45°C for 1 month.

After washing the hands and forearms with the compositions made consistent with the invention, all trained panelists concluded that the wash compositions rinsed off easily, left a filmless sensation and lathered well. Also, the panelists surprisingly concluded the compositions made according to the invention performed similar to those made with traditional polymeric synthetic thickeners.

### Example IV

The biodegradable thickeners identified in TABLE VI were used in combination with wash compositions similar to those identified in Example I, except that the surfactant system used was consistent with wash composition 1 and only unsaturated betaine was used as the zwitterionic surfactant.

**Table VI**

| Starch | Cellulose | Iota Carageenan | Viscosity, mPas | Stability |
|---|---|---|---|---|
| 3.5 | 0.75 | | 10930 | Stable |
| 3.5 | 2.5 | | 9204 | Stable |
| 4.3 | 1.0 | | 6749 | Stable |
| 4.5 | 0.5 | | 3098 | Stable |
| 4.5 | 1.0 | | 15990 | Stable |
| 4.5 | 2.0 | | 15020 | Stable |
| 5.0 | 0.75 | | 12250 | Stable |
| 5.0 | 2.5 | | 17650 | Stable |
| 3.5 | | 0.5 | 7613 | Stable |
| 3.5 | | 0.65 | 8113 | Stable |
| 3.5 | | 0.75 | 7134 | Stable |
| 4.3 | | 0.5 | 8142 | Stable |
| 4.3 | | 0.65 | 9091 | Stable |
| 4.5 | | 0.5 | 9008 | Stable |

The wash compositions were stored at elevated temperatures, not exceeding 50°C. In less than one week, compositions not made according to the present invention (i.e., not comprising unsaturated zwitterionic as herein described) discolored and separated and were not usable wash compositions. In contrast, the wash compositions made according to the invention were stable, did not discolor, and maintained viscosity even after being stored at 45°C for 1 month.

After washing the hands and forearms with the compositions made consistent with the invention, all trained panelists concluded that the wash compositions rinsed off easily, left a filmless sensation and lathered well. Also, the panelists surprisingly concluded the compositions made according to the invention performed similar to those made with traditional polymeric synthetic thickeners.

## Claims

1. A wash composition comprising:
a) at least one anionic surfactant, wherein the anionic surfactant comprises a glutamate, glycinate, taurate, isethionate or mixture thereof;
b) unsaturated zwitterionic surfactant;
c) 0 to 2% by weight nonionic surfactant; and
d) thickener having starch, and at least one of cellulose and gum, wherein the gum selected from the group consisting of hydroxypropyltrimethyl ammonium chloride guar, locust bean gum, konjac mannan gum, gum tragacanth, sodium or propylene glycol alginate, kappa-, iota-, or lambda-carrageenan, guar, hydroxylpropyl guar gum, karaya gum, gum arabic, gellan, xanthan, succinoglycan or its acidic or enzymatic hydrolysates, agar, pectin, and mixtures thereof,
wherein the unsaturated zwitterionic surfactant comprises a hydrophobic tail comprising 2.5 to 20% by weight C18:1, and 0.45 to 4.5%, by weight C18:2, and 5 to 22% by weight in total of C₈ and C₁₀ carbon chains based on total weight of the hydrophobic tail on the zwitterionic surfactant.

2. The wash composition according to claim 1, wherein the unsaturated zwitterionic surfactant comprises unsaturated betaine, sultaine or both.

3. The wash composition according to claim 2, wherein the C₈ and C₁₀ hydrophobic tails are present at a weight ratio from 3:7 to 7:3.

4. The wash composition according to any of the preceding claims wherein the thickener consists of organic matter.

5. The wash composition according to claim 4, wherein the thickener can be broken down by microorganisms.

6. The wash composition according to any of the preceding claims wherein the unsaturated zwitterionic 3.5 to 15% by weight C18:1, and 0.75 to 4% by weight C18:2, and 5 to 22%, and 6.5 to 20% by weight in total of C₈ and C₁₀ carbon chains based on total weight of hydrophobic tail on the zwitterionic surfactant.

7. The wash composition according to any of the preceding claims wherein the C₈ and C₁₀ hydrophobic tails are present at a weight ratio from 4:6 to 6:4.

8. The wash composition according to any of the preceding claims wherein the composition is substantially free of sulfate and soap, wherein "substantially free" means less than 3.5%, and preferably, less than 2.5%, more preferably, less than 1.25%, and most preferably, less than 0.65% by weight of the of the wash composition.

9. The wash composition according to any of the preceding claims wherein the composition comprises less than 0.35% by weight synthetic thickener, wherein the synthetic thickener is an ammonium acryloyldimethyltaurate vinylpyrrolidone copolymer and/or alkyl acrylate cross polymers.

10. The wash composition according to any of the preceding claims wherein the composition is isotropic or lamellar.

11. The wash composition according to any of the preceding claims wherein the composition further comprises thiamidol,12-hydroxystearic acid, niacinamide terpineol, thymol or a mixture thereof.

12. The wash composition according to any of the preceding claims wherein the composition has a pH from 5.25 to 7.75.

13. The wash composition according to any one of the preceding claims, wherein the anionic is a taurate, isethionate, glycinate, glutamate or a mixture thereof.

14. The wash composition according to any of the preceding claims wherein the composition is substantially free of hydantoins, wherein "substantially free" means less than 3.5%, and preferably, less than 2.5%, more preferably, less than 1.25%, and most preferably, less than 0.65% by weight of the of the wash composition.

## Patentansprüche

1. Waschmittelzusammensetzung, umfassend:
a) mindestens ein anionisches Tensid, wobei das anionische Tensid ein Glutamat, Glycinat, Taurat, Isethionat oder eine Mischung davon umfasst;
b) ein ungesättigtes zwitterionisches Tensid;
c) 0 bis 2 Gew.-% nichtionisches Tensid; und
d) ein Verdickungsmittel mit Stärke und mindestens einem der Stoffe Cellulose und Gummi, wobei das Gummi aus der Gruppe ausgewählt ist, bestehend aus Hydroxypropyltrimethylammoniumchlorid-Guar, Johannisbrotkernmehl, Konjakmannan-Gummi, Tragantgummi, Natrium- oder Propylenglykolalginat, Kappa-, Iota- oder Lambda-Carrageen, Guar, Hydroxypropylguargummi, Karaya-Gummi, Gummi arabicum, Gellan, Xanthan, Succinoglykan oder dessen sauren oder enzymatischen Hydrolysaten, Agar, Pektin und Mischungen davon, wobei das ungesättigte zwitterionische Tensid einen hydrophoben Schwanz umfasst, der 2,5 bis 20 Gew.-% C18:1 und 0,45 bis 4,5 Gew.-% C18:2 und insgesamt 5 bis 22 Gew.-% C₈- und C₁₀-Kohlenstoffketten, bezogen auf das Gesamtgewicht des hydrophoben Schwanzes des zwitterionischen Tensids, umfasst.

2. Waschmittelzusammensetzung nach Anspruch 1, wobei das ungesättigte zwitterionische Tensid ungesättigtes Betain, Sultain oder beides umfasst.

3. Waschmittelzusammensetzung nach Anspruch 2, wobei die hydrophoben C₈- und C₁₀-Schwänze in einem Gewichtsverhältnis von 3:7 bis 7:3 vorliegen.

4. Waschmittelzusammensetzung nach einem der vorhergehenden Ansprüche, wobei das Verdickungsmittel aus organischem Material besteht.

5. Waschmittelzusammensetzung nach Anspruch 4, wobei das Verdickungsmittel durch Mikroorganismen abgebaut werden kann.

6. Waschmittelzusammensetzung nach einem der vorhergehenden Ansprüche, wobei das ungesättigte zwitterionische Tensid 3,5 bis 15 Gew.-% C18:1 und 0,75 bis 4 Gew.-% C18:2 und 5 bis 22 Gew.-% und insgesamt 6,5 bis 20 Gew.-% C₈- und C₁₀-Kohlenstoffketten, bezogen auf das Gesamtgewicht des hydrophoben Schwanzes des zwitterionischen Tensids umfasst.

7. Waschmittelzusammensetzung nach einem der vorhergehenden Ansprüche, wobei die hydrophoben C₈- und C₁₀-Schwänze in einem Gewichtsverhältnis von 4:6 bis 6:4 vorliegen.

8. Waschmittelzusammensetzung nach einem der vorhergehenden Ansprüche, wobei die Zusammensetzung im Wesentlichen frei von Sulfat und Seife ist, wobei "im Wesentlichen frei" weniger als 3,5 Gew.-% und vorzugsweise weniger als 2,5 Gew.-%, bevorzugter weniger als 1,25 Gew.-% und höchst bevorzugt weniger als 0,65 Gew.-% der Waschmittelzusammensetzung bedeutet.

9. Waschmittelzusammensetzung nach einem der vorhergehenden Ansprüche, wobei die Zusammensetzung weniger als 0,35 Gew.-% synthetisches Verdickungsmittel umfasst, wobei das synthetische Verdickungsmittel ein Ammoniumacryloyldimethyltaurat-Vinylpyrrolidon-Copolymer und/oder Alkylacrylat-Crosspolymer ist.

10. Waschmittelzusammensetzung nach einem der vorhergehenden Ansprüche, wobei die Zusammensetzung isotrop oder lamellar ist.

11. Waschmittelzusammensetzung nach einem der vorhergehenden Ansprüche, wobei die Zusammensetzung ferner Thiamidol, 12-Hydroxystearinsäure, Niacinamid, Terpineol, Thymol oder eine Mischung davon umfasst.

12. Waschmittelzusammensetzung nach einem der vorhergehenden Ansprüche, wobei die Zusammensetzung einen pH-Wert von 5,25 bis 7,75 aufweist.

13. Waschmittelzusammensetzung nach einem der vorhergehenden Ansprüche, wobei das anionische Tensid ein Taurat, Isethionat, Glycinat, Glutamat oder eine Mischung davon ist.

14. Waschmittelzusammensetzung nach einem der vorhergehenden Ansprüche, wobei die Zusammensetzung im Wesentlichen frei von Hydantoinen ist, wobei "im Wesentlichen frei" weniger als 3,5 Gew.-% und vorzugsweise weniger als 2,5 Gew.-%, bevorzugter weniger als 1,25 Gew.-% und höchst bevorzugt weniger als 0,65 Gew.-% der Waschmittelzusammensetzung bedeutet.

## Revendications

1. Composition de lavage comprenant :
a) au moins un tensioactif anionique, dans laquelle le tensioactif anionique comprend un glutamate, glycinate, taurate, iséthionate ou mélange de ceux-ci ;
b) un tensioactif zwittérionique insaturé ;
c) 0 à 2 % en poids de tensioactif non-ionique ; et
d) un épaississant ayant de l'amidon, et au moins l'une parmi la cellulose et une gomme, dans lequel la gomme est choisie dans le groupe constitué par le chlorure d'hydroxypropyltriméthylammonium-guar, la gomme de caroube, la gomme de mannane de konjac, la gomme adragante, l'alginate de sodium ou de propylèneglycol, le carraghénane kappa, iota ou lambda, le guar, la gomme d'hydroxypropyl-guar, le gomme karaya, la gomme arabique, la gellane, le xanthane, le succinoglycane ou ses hydrolysats acides ou enzymatiques, la gélose, la pectine, et des mélanges de ceux-ci,
dans laquelle le tensioactif zwittérionique insaturé comprend une queue hydrophobe comprenant 2,5 à 20 % en poids de C18:1, et 0,45 à 4,5 % en poids de C18:2, et 5 à 22 % en poids au total de chaînes carbonées en C₈ et C₁₀, par rapport au poids total de la queue hydrophobe sur le tensioactif zwittérionique.

2. Composition de lavage selon la revendication 1, dans laquelle le tensioactif zwittérionique insaturé comprend une bétaïne insaturée, une sultaïne, ou les deux.

3. Composition de lavage selon la revendication 2, dans laquelle les queues hydrophobes en C₈ et C₁₀ sont présentes en un rapport en poids de 3/7 à 7/3.

4. Composition de lavage selon l'une quelconque des revendications précédentes, dans laquelle l'épaississant est constitué de matière organique.

5. Composition de lavage selon la revendication 4, dans laquelle l'épaississant peut être désagrégé par des microorganismes.

6. Composition de lavage selon l'une quelconque des revendications précédentes, dans laquelle le zwittérionique insaturé comprend 3,5 à 15 % en poids de C18:1, et 0,75 à 4 % en poids de C18:2, et 5 à 22 %, et 6,5 à 20 % en poids au total de chaînes carbonées en C₈ et C₁₀, par rapport au poids total de la queue hydrophobe sur le tensioactif zwittérionique.

7. Composition de lavage selon l'une quelconque des revendications précédentes, dans laquelle les queues hydrophobes en C₈ et C₁₀ sont présentes en un rapport en poids de 4/6 à 6/4.

8. Composition de lavage selon l'une quelconque des revendications précédentes, dans laquelle la composition est pratiquement exempte de sulfate et de savon, dans laquelle "pratiquement exempte" signifie moins de 3,5 %, et de préférence moins de 2,5 %, mieux encore moins de 1,25 %, et tout spécialement moins de 0,65 % en poids de la composition de lavage.

9. Composition de lavage selon l'une quelconque des revendications précédentes, dans laquelle la composition comprend moins de 0,35 % en poids d'épaississant synthétique, dans laquelle l'épaississant synthétique est un copolymère d'acryloyldiméthyltaurate d'ammonium et de vinylpyrrolidone et/ou un polymère réticulé d'acrylate d'alkyle.

10. Composition de lavage selon l'une quelconque des revendications précédentes, dans laquelle la composition est isotrope ou lamellaire.

11. Composition de lavage selon l'une quelconque des revendications précédentes, dans laquelle la composition comprend en outre du thiamidol, de l'acide 12-hydroxystéarique, du niacinamide, du terpinéol, du thymol ou un mélange de ceux-ci.

12. Composition de lavage selon l'une quelconque des revendications précédentes, dans laquelle la composition a un pH de 5,25 à 7,75.

13. Composition de lavage selon l'une quelconque des revendications précédentes, dans laquelle l'anionique est un taurate, iséthionate, glycinate, glutamate, ou un mélange de ceux-ci.

14. Composition de lavage selon l'une quelconque des revendications précédentes, dans laquelle la composition est pratiquement exempte d'hydantoïnes, dans laquelle "pratiquement exempte" signifie moins de 3,5 %, et de préférence moins de 2,5 %, mieux encore moins de 1,25 %, et tout spécialement moins de 0,65 % en poids de la composition de lavage.
